# EUROPÄISCHE PATENTSCHRIFT

(11) **EP 1 168 989 B1**
(45) Veröffentlichungstag und Bekanntmachung des Hinweises auf die Patenterteilung: **15.09.2004**
(21) Anmeldenummer: 00926938.2
(22) Anmeldetag: 14.04.2000
(51) Int. Cl.: A61F 2/36, A61B 17/16, A61B 17/17

(54) **GELENKKOPFPROTHESE UND BAUSATZ ZUR BILDUNG EINER SOLCHEN**
CONDYLE PROSTHESIS AND KIT FOR PRODUCING SAME
PROTHESE DE TETE D'ARTICULATION ET PIECES NECESSAIRES A SA REALISATION

(30) Priorität: 15.04.1999 CH 69399
(43) Veröffentlichungstag der Anmeldung: 09.01.2002
(73) Patentinhaber: Sutter, Franz, 4435 Niederdorf (CH)
(72) Erfinder: Sutter, Franz, 4435 Niederdorf (CH)
(74) Vertreter: Böhme, Ulrich, Dr. Dipl.-Phys.
(86) Internationale Anmeldenummer: PCT/EP2000/003392
(87) Internationale Veröffentlichungsnummer: WO 2000/062718

(56) Entgegenhaltungen:
- EP-A- 0 094 829
- EP-A- 0 363 019
- EP-A- 0 485 326
- WO-A-91/07932
- WO-A-93/16663
- WO-A-96/07374
- DE-A- 3 006 178
- FR-A- 2 353 275
- FR-A- 2 578 738
- FR-A- 2 631 543
- FR-A- 2 674 122
- US-A- 2 682 265
- US-A- 4 846 841
- US-A- 5 376 126
- US-A- 5 571 203

## Beschreibung

Die Erfindung betrifft eine Gelenkkopfprothese zur Befestigung an einem Knochen, insbesondere einem Femur, mit einem einen ringförmigen Abschnitt aufweisenden Träger, einem ursprünglich separaten, fest mit diesem verbindbaren, eine kugelkalottenförmige Gleitfläche bildenden Gelenkkappe und mit mindestens einem vom Träger und von der Gleitfläche wegragenden hülsenförmigen Verankerungselement mit einer geraden Achse, wobei der Träger einen von mindestens einem Verankerungselement wegragenden Vorsprung, und die Gelenkkappe ein Sackloch aufweisen, in welches der Vorsprung bei zusammengesetzter Gelenkkopfprothese hineinragt und die Gelenkkappe festhält.

Bekannte Gelenkkopfprothesen für Hüftgelenke besitzen einen vollständigen, kugelförmigen Kopf und einen starr mit diesem verbundenen, abgewinkelten oder abgebogenen Dorn, wobei der Dorn im wesentlichen einen vollen Querschnitt hat. Zum Befestigen einer solchen Prothese an einem Femur werden der natürliche Gelenkkopf sowie ein Teil des Halses des Femurs abgeschnitten und ein Loch in den Schaft des Femurs gebohrt, so daß viel Knochenmaterial entfernt werden muß und viele Blutgefäße zerstört werden. Zudem hat der abgewinkelte oder abgebogene metallische, normalerweise in den Femur-Schaft einzementierte Dorn der Prothese eine viel größere Biegesteifigkeit als ein natürlicher Femur. Dies ergibt den Nachteil, daß an den Dorn angrenzende Bereiche des Femurs und eventuell auch andere Körperteile bei Belastungen häufig stellenweise viel stärker und/oder stellenweise viel weniger stark beansprucht werden als bei entsprechenden Belastungen eines vollständig natürlichen Femurs und daß dadurch die Langzeitstabilität der Verbindung der Prothese mit dem Femur beeinträchtigt werden kann.

Aus den CH-A 642 250 und CH-A 642 251 sowie aus der WO-A-96/07374 sind nun Gelenkkopfprothesen bekannt, die eine Gelenkkappe besitzen, die direkt am Schenkelhals verankert ist. Diese Prothesen weisen einen oder mehrere Dorne als Verankerungselemente auf, die zum Beispiel aus einer zylindrischen Hülse mit einem perforierten Mantel bestehen. Diese Dorne werden in hohlzylinderförmigen Bohrungen im Femur eingebracht und verwachsen mit dem Knochen, so daß es nicht nötig ist, die Prothese mittels Knochenzement zu befestigen. Prothesen dieser Art weisen den Vorteil auf, daß nur wenig Knochenmaterial entfernt werden muß und ein großer Teil des natürlichen Knochens erhalten bleibt. Das hat unter anderem zur Folge, daß die Biegesteifigkeit des mit der Gelenkkopfprothese versehenen Femurs praktisch gleich ist wie die eines Femurs mit einem natürlichen Gelenkkopf.

Die aus der CH-A 642 250, der CH-A 642 251 und aus der WO-A-96/07374 bekannten Gelenkkopfprothesen weisen einen Gelenkkopf auf, dessen kugelkalottenförmige Gleitfläche einen Kugeldurchmesser definiert, der ungefähr demjenigen des zu ersetzenden natürlichen Gelenkkopfes entspricht und typischerweise ungefähr 42 bis 48 mm beträgt. Aufgrund der beschränkten Dicke des Hüftbeins hat das zur Folge, daß ein üblicherweise aus Polyethylen bestehendes, auf den Gelenkkopf passendes künstliches Acetabulum nur eine beschränkte Dicke von typischerweise ungefähr 4 mm aufweisen kann. Das ist deshalb ungünstig, weil das Acetabulum dementsprechend schwach ist und sich durch Reibung zwischen der üblicherweise aus Titan, Kobalt oder Keramik bestehenden Gleitfläche des Gelenkkopfes und dem Acetabulum im Lauf der Zeit eine verhältnismäßig starke Abnutzung ergeben kann, so daß das Acetabulum nur eine relativ kurze Lebensdauer hat. Damit das Acetabulum im verfügbaren Raum dicker gemacht werden kann, sollte der Kugeldurchmesser bei der Gelenkkopfprothese kleiner bemessen werden als bei einem natürlichen Gelenk. Bei den genannten, bekannten Prothesen würde eine Verkleinerung des Kugeldurchmessers jedoch die Stabilität der Verbindung der Prothese mit dem Knochen schwächen und hätte zudem den Verlust von viel Knochenmaterial und von vielen Blutgefäßen zur Folge. Dies ist insbesondere dann der Fall, wenn das Kugelzentrum bei der Prothese ungefähr die gleiche Lage in Bezug auf den Knochenschaft einnehmen soll wie beim natürlichen Gelenk.

Eine weitere Problemstellung ergibt sich in der Anpassung der genauen Gelenkposition an die Anatomie der Patientin oder des Patienten. Es ist von Vorteil, wenn ein künstliches Hüftgelenk so bemessen und angebracht ist, daß sich das Zentrum der Gelenkkugel nach der Operation an derselben Stelle befindet wie beim ersetzten natürlichen Gelenk. Bei bekannten Gelenkkopfprothesen wird dieses Problem angegangen, indem das bestehende Hüftgelenk der Patientin oder des Patienten möglichst genau vermessen und die Prothese diesen Maßen angepaßt wird. Wenn sich jedoch bei oder nach der Operation irgendwelche unvorhergesehene Verschiebungen ergeben oder wenn die Vermessung zuwenig genau erfolgt ist, wird eine erneute Anpassung sehr schwierig sein, da das Auswechseln einer Gelenkprothese mit einer äußerst aufwendigen Operation verbunden ist und für jede neue Operation meistens weitere Teile des Femurs entfernt werden müssen.

Aus der US-A-4 846 841 ist eine Gelenkkopfprothese bekannt, die einen auf dem Stumpf eines Femurs zu befestigenden Träger und eine auf diesem befestigbare, als Gelenkkopf dienende Schale aufweist. Durch Abstimmen der Form der Schale kann beispielsweise der Außendurchmesser der Gleitfläche innerhalb eines gewissen Bereiches notfalls durch Auswechseln der Schale angepaßt werden. Die in der US-4 846 841 offenbarte Gelenkkopfprothese weist ansonsten jedoch die gleichen Nachteile auf wie die bereits erwähnten, aus der CH-A 642 250, der CH-A 642 251 und aus der WO-A-96/07374 bekannten Gelenkkopfprothesen. Zusätzlich muß, damit der Träger aufgesetzt werden kann, der Stumpf des Femurs in eine zylindrische Form gebracht werden, was die Verankerung der Prothese nicht ideal löst und weshalb wichtige Blutbahnen unterbrochen werden müssen. Auch ist die Krafteinleitung bei der in der US-4 846 841 offenbarten Art der Verankerung nicht ideal gelöst, da bei seitlich am Gelenkkopf angreifenden Kräften die Gefahr der Abscherung des Knochenstumpfes besteht.

Ein zusätzlicher Nachteil von Gelenkkopfprothesen, die direkt am Schenkelhals verankert werden, ergibt sich für Hüftgelenkpatienten, die zusätzlich beispielsweise an Osteoporose leiden. Bei solchen Patientinnen oder Patienten neigt der Schenkelhals dazu, bei größeren Belastungen zu brechen, was zusätzliche komplizierte Operationen nötig macht und dazu führt, daß die Prothese durch eine am Schenkelschaft ansetzende konventionelle Prothese mit den vorstehend beschriebenen Nachteilen ersetzt werden muß.

Aus der DE-A-28 45 231 ist nun ein Implantierungsverfahren zum Befestigen eines Prothesenteils an einem Knochen bekannt, das unter anderem vorsieht, daß ein Loch vom Kopf des Oberschenkelhalses bis zur gegenüberliegenden Seite des Oberschenkelknochens durchgebohrt wird, und in dieses ein Schaft eingebracht wird, an dem der künstliche Gelenkkopf befestigt wird. Das soll unter anderem bewirken, daß der Oberschenkelhals weniger leicht bricht. Das in der DE-A-28 45 231 offenbarte Implantierungsverfahren sieht hingegen das Anbringen des Gelenkkopfes nur bei einem noch intakten Schenkelhals vor und bietet keine Lösung an für Patientinnen oder Patienten, die bereits eine Fraktur erlitten haben. Auch ist nicht vorgesehen, daß der Schaft mit Mitteln versehen ist, durch die er mit dem Knochen verwachsen kann, was die Wirksamkeit der Frakturprävention erheblich einschränkt. Außerdem können mit diesem Implantierungsverfahren nur Gelenkkopfprothesen am Femur angebracht werden, die einen Gelenkkopf aufweisen, dessen Durchmesser gleich groß ist wie derjenige des ersetzten natürlichen Gelenkkopfes.

Eine gattungsgemäße Gelenkkopfprothese ist in der WO 93/16663 beschrieben. Diese weist eine ebene Auflagefläche auf mit einem kompakten, stiftförmigen Verankerungselement. Eine ähnliche Prothese zeigt die WO-A-91/07932, auch bei dieser Prothese ist eine ebene Auflagefläche vorgesehen, so daß in beiden Fällen der verbleibende Knochenstumpf sehr weit reseziert werden muß.

Ausgehend von diesem Stand der Technik liegt dem Anmeldegegenstand die Aufgabe zugrunde, eine Gelenkprothesezu schaffen, die es ermöglicht, die Substanz des Knochens weitgehend zu erhalten, so daß dadurch auch quer zur Schenkelhalsachse verlaufende Kraftkomponenten auf den Knochen übertragen werden können.

Diese Aufgabe wird bei einer Gelenkkopfprothese der eingangs beschriebenen Art erfindungsgemäß dadurch gelöst, daß der Träger eine Auflagefläche mit einem wannenförmigen Abschnitt aufweist, den der ringförmige Abschnitt umschließt. Durch das Vorsehen eines wannenförmigen Abschnittes kann die Abtragung des Femurstumpfes auf ein Minimum beschränkt werden, und dieser Stumpf taucht derart in den wannenförmigen Abschnitt ein, das auch quer verlaufende Kraftkomponenten von der Prothese auf den Knochen übertragen werden können.

Vorteilhafte Ausgestaltungen des Erfindungsgegenstandes gehen aus den abhängigen Ansprüchen hervor.

Eine erfindungsgemäße Gelenkkopfprothese weist verschiedene Vorteile auf. Dadurch, daß der Femur bei einer Bestückung mit einer erfindungsgemäßen Prothese weitgehend erhalten werden kann, ist auch die Biegesteifigkeit weitgehend die gleiche wie bei einem Knochen mit einem natürlichen Gelenk. Der von der kalottenförmigen Gleitfläche definierte Kugeldurchmesser kann bei der erfindungsgemäßen Prothese ohne weiteres kleiner bemessen werden als der entsprechende Durchmesser des ersetzten natürlichen Gelenkkopfes. Der Kugeldurchmesser der genannten Gleitfläche kann bei einer Prothese für einen erwachsenen Menschen vorzugsweise mindestens 20 mm und höchstens 40 mm und zum Beispiel 30 mm bis 38 mm betragen. Das Acetabulum kann dann dicker ausgebildet werden als bei Gelenkprothesen, bei denen der Kugeldurchmesser ungefähr gleich dem Durchmesser des natürlichen Gelenkkopfes ist. Die charakteristische Dicke des Acetabulums kann dann mindestens 6 mm bis etwa 10 mm oder eventuell noch mehr betragen. Das Acetabulum besitzt dann auch bei einer Ausbildung aus einem Kunststoff, beispielsweise Polyethylen, eine ausreichende Festigkeit sowie eine hohe Lebensdauer.

Dadurch, daß die Gelenkkopfprothese einen Träger und eine aufsetzbare Gelenkkappe aufweist, kann beispielsweise der Abstand zwischen dem Femur und dem Hüftbein über die Abmessungen der Gelenkkappe eingestellt werden. Zusätzlich läßt sich auch die laterale Position der Gelenkkappe einfach variieren und den Belastungsverhältnissen anpassen. Dadurch, daß die Gelenkkappe lediglich auf den Träger aufgesetzt ist, kann sie bei Bedarf auch mit einer verhältnismäßig einfachen Operation ausgewechselt werden, zum Beispiel wenn sie abgenützt ist, oder wenn ihre Position erneut angepaßt werden muß.

Die Erfindung betrifft auch einen Bausatz zur Bildung einer Gelenkkopfprothese gemäß Anspruch 28. Der Bausatz weist einen Träger und mindestens zwei Gelenkkappen auf, die sich beispielsweise in relativen lateralen oder axialen Positionen des Gelenkkopfes im Bezug auf den Träger unterscheiden, aus denen der behandelnde Arzt je nach Erfordernis auswählen und die er ohne chirurgische Eingriffe am Femur austauschen kann.

Auch aus Kostengründen ist die erfindungsgemäße Ausgestaltung eines Kugelgelenkes interessant. Durch das einfache Aufsetzen der Gelenkkappe, wobei nur eine grobe Anpassung an den Femur des Patienten erfolgen muß, wird nämlich die Verwendung von Gelenkkappen in Standardgrößen möglich.

Im folgenden werden Ausführungsbeispiele der Erfindung anhand einer Zeichnung beschrieben. In der Zeichnung zeigt
- die Figur 1: einen Schnitt durch einen Femur, der mit einer Ausführungsform einer erfindungsgemäßen Gelenkkopfprothese versehen ist, die in ein ebenfalls künstliches Acetabulum eines Hüftbeins eingesetzt ist,
- die Figur 2: eine Explosionsdarstellung eines Femurs mit der Gelenkkopfprothese aus Figur 1,
- die Figur 3: einen teilweisen Schnitt durch einen Femur und einen Fräser zum Fräsen der AuBenfläche des Gelenkkopfes,
- die Figur 4: einen teilweisen Schnitt durch einen Femur und ein Werkzeug zum Fräsen eines Ringspaltes zum Aufnehmen einer Hülse der Prothese,
- die Figur 5: einen Schnitt durch einen mit einer erfindungsgemäßen Gelenkkopfprothese ausgestatteten Femur mit einer schematisch dargestellten Zug- und Druckliniencharakteristik,
- die Figur 6: ein Diagramm, in dem die Auslenkung der Gelenkköpfe eines natürlichen Femurs und eines mit einer nicht erfindungsgemäßen Schaftprothese versehenen Femurs in Funktion einer an diesen angreifenden Kraft aufgetragen ist,
- die Figur 7: eine Darstellung eines mit einer erfindungsgemäßen Gelenkkopfprothese ausgestatteten Femurs, aus der ersichtlich ist, wie durch verschiedene Abmessungen des Kopfes der Abstand zwischen Hüftbein und Femur variiert werden kann,
- die Figur 8: eine schematische Darstellung eines mit einer erfindungsgemäßen Gelenkkopfprothese ausgestatteten Femurs, aus der ersichtlich ist, wie durch verschiedene Abmessungen des Kopfes seine laterale Position variiert werden kann,
- die Figur 9: eine Aufsicht auf eine Variante für die Ausgestaltung eines Verankerungselementes, und
- die Figur 10: eine Explosionsdarstellung eines Femurs mit einer anderen Ausführungsform einer erfindungsgemäßen Gelenkkopfprothese.

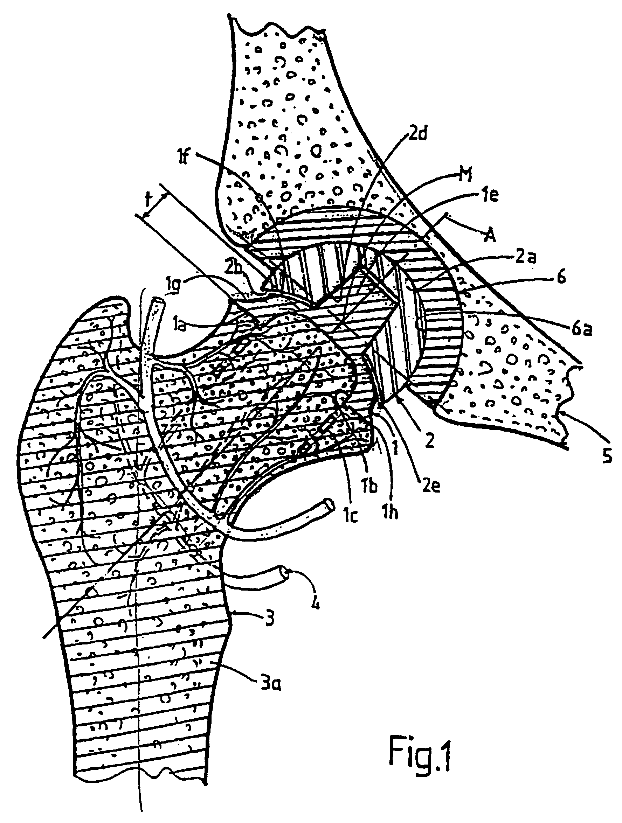

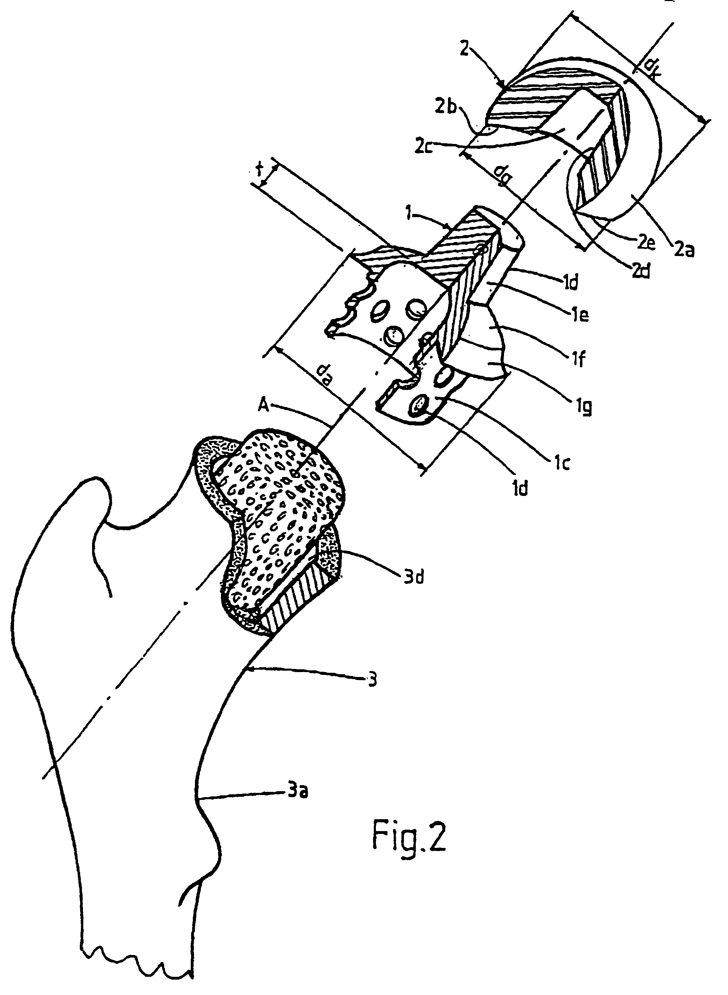

Eine erste bevorzugte Ausführungsform der Erfindung ist in den Figuren 1 und 2 der Zeichnung dargestellt. Die Gelenkkopfprothese besitzt einen Träger 1 und eine ursprünglich separate Gelenkkappe 2. Die Gelenkkappe 2 besteht vorzugsweise aus Keramik, beispielsweise Zirkoniumoxyd, kann jedoch auch aus Aluminiumoxyd, Metalllegierung ober bei Verwendung in Kombination mit einem Metall-Acetabulum auch aus Kunststoff hergestellt sein. Sie hat im wesentlichen die Form einer Kugel mit Mittelpunkt M und Durchmesser dₖ. Sie weist eine glatt polierte kalottenförmige und als Gleitfläche 2a dienende Fläche auf, die eine Kopfachse A definiert, bezüglich der sie rotationssymmetrisch ist. Die Oberfläche der Gelenkkappe 2 weist weiter einen an die Gleitfläche 2a anschließenden Abschnitt 2b auf, der rotationssymmetrisch und konkav, konisch oder eventuell konvex ausgeformt sein kann und ebenfalls glatt poliert ist. Weiter besitzt die Gelenkkappe ein Sackloch 2c, das durch eine Innenfläche 2d begrenzt wird und das beispielsweise konisch sein kann.

Der Träger 1 ist ebenfalls rotationssymmetrisch bezüglich der Kopfachse A. Er weist eine Auflagefläche auf, die dazu bestimmt ist, auf dem Femur 3 aufzuliegen und die unterteilt ist in eine äußere Auflagefläche 1a und eine innere Auflagefläche 1b. Die äußere Auflagefläche 1a ist im wesentlichen ringförmig mit einem Rand 1h, der einen Kreis mit einem Durchmesser dₐ bildet. Die Breite des ringförmigen Abschnittes ist dabei, in einer zur Kopfachse A senkrechten Ebene gemessen, im allgemeinen mindestens 12 % und vorzugsweise 12 bis 30 % vom dₐ. Die innere Auflagefläche 1b, die an die äußere Auflagefläche anschließt, hat die Form einer Wanne. Die parallel zur Kopfachse A gemessene Tiefe der Wanne beträgt dabei mindestens 12 %, vorzugsweise aber 15 bis 25 % des Durchmessers dₐ des Randes 1h der äußeren Auflagefläche 1a, das heißt, wenn die Prothese für den Femur 3 eines erwachsenen normalwüchsigen Menschen bestimmt ist, ist die Tiefe t der Wanne somit mindestens 5 mm und vorzugsweise ca. 6 bis 10 mm.

Zusätzlich weist der Träger 1 auch noch ein Verankerungselement 1c auf, das als Hohlzylinder mit Perforation 1d ausgebildet ist. Die Achse des Hohlzylinders fällt dabei im zusammengesetzten Zustand mit der Kopfachse A zusammen.

Weiter weist der Träger 1 einen Vorsprung 1e auf. Der Vorsprung 1e ist als Konus ausgebildet, wobei die Konusfläche mit der Achse des Konus einen Winkel von höchstens 10' bildet. Im zusammengesetzten Zustand ragt der Vorsprung 1e in das Sackloch 2c der Gelenkkappe 2 hinein, so daß die Konusachse mit der Kopfachse zusammenfällt und der Vorsprung 1e die Gelenkkappe 2 festhält, so daß Träger 1 und Gelenkkappe 2 starr verbunden sind. Zusätzlich besitzt der Träger noch eine als Schulterfläche ausgebildete Außenfläche 1f und eine weitere Außenfläche 1g, die beide ebenfalls rotationssymmetrisch bezüglich A sind, wobei die Außenfläche 1g einen äußeren Durchmesser dₐ aufweist, der größer ist als der Durchmesser d_{g} des Kreises, der den Rand 2e der Gleitfläche 2a bildet. Die Außenfläche 1g kann dabei konkav sein, wie das in den Figuren 1 und 2 dargestellt ist, aber auch eine andere Ausformung, zum Beispiel eine konische oder sphärische Form ist denkbar.

Der Träger kann auch ohne eine als Schulterfläche ausgebildete Außenfläche 1f ausgeführt sein.

Die Gelenkkopfprothese ist so dimensioniert, daß der Durchmesser dₖ der Kugel, die von der Gleitfläche 2a definiert wird, kleiner ist als der Durchmesser dₐ des Auflageflächenrandes 1h. Er beträgt 20 bis 40 mm, vorzugsweise 30 bis 38 mm, oder in relativen Maßen ausgedrückt ca. 50 % bis 90 % und vorzugsweise ca. 65 % bis 85 % von dₐ.

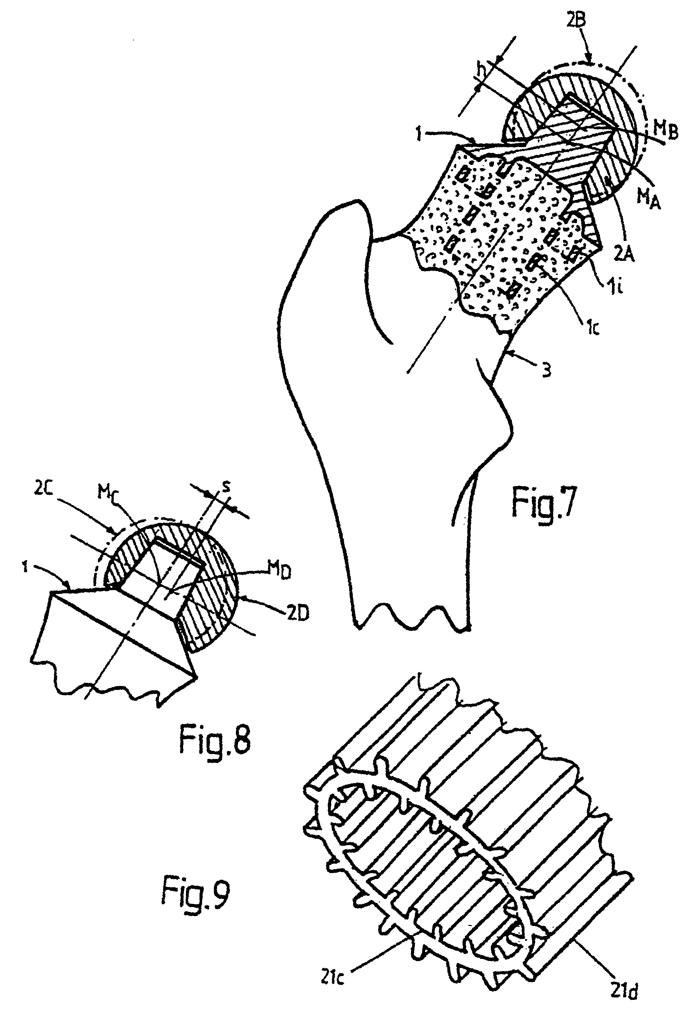

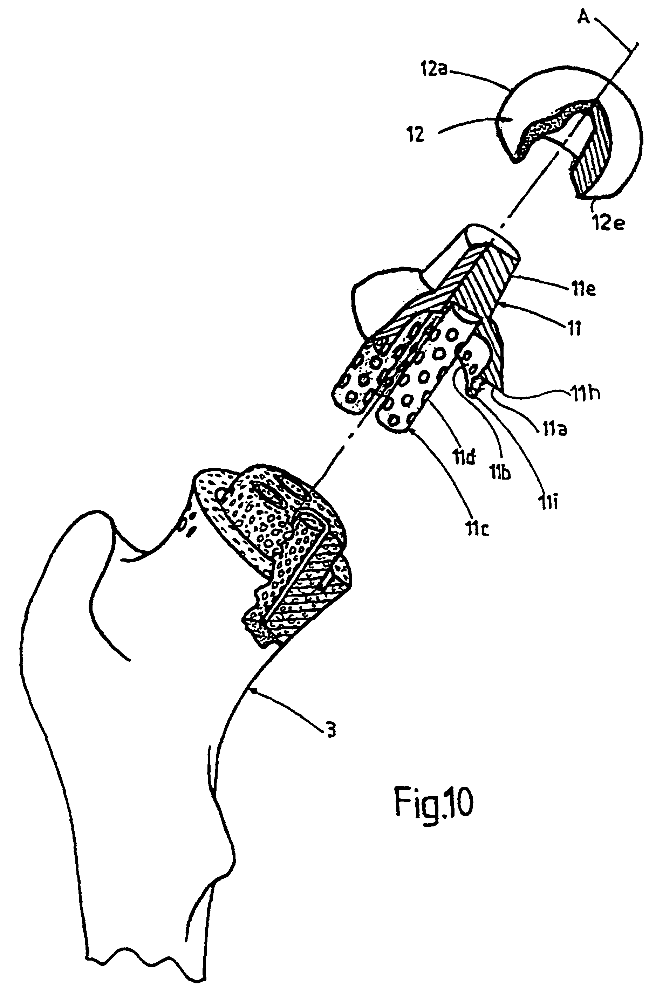

Eine andere Ausführungsform der Erfindung ist in der Figur 10 dargestellt. Die Prothese weist ebenfalls einen Träger 11 und eine Gelenkkappe 12 auf. Der Träger 11 besitzt eine äußere Auflagefläche 11a und eine innere Auflagefläche 11b. Die in der Figur 10 dargestellte Prothese unterscheidet sich von der in den Figuren 1 und 2 dargestellten dadurch, daß an ihrer inneren Auflagefläche 11b mehrere, beispielsweise drei Verankerungselemente 11c mit zur Kopfachse A parallel verlaufenden Achsen ansetzen, die ebenfalls als Hülsen mit Perforationen 11d ausgebildet sind. Je nach Bedarf und je nach Zustand des Femurstumpfes, auf den die Prothese aufgebracht werden soll, kann die Anordnung der Verankerungselemente 11c dabei symmetrisch oder asymmetrisch bezüglich der Kopfachse A geschehen. Ein Stabilisierungsring 11i dient der zusätzlichen Stabilität der Verbindung zwischen Femur 3 und Träger 11, ein solcher Stabilisierungsring 1i ist übrigens auch bei dem Ausführungsbeispiel-der Figur 7 vorgesehen.

Eine andere Version eines Verankerungselementes 21c ist in der Figur 9 dargestellt, das Verankerungselement 21c ist ebenfalls hohlzylinderförmig, weist aber anstelle der Perforationen 1d, 11d oder zusätzlich zu den Perforationen 1d, 11d Vorsprünge 21d nach innen und außen auf, die dazu bestimmt sind, ins Innere des Knochens 3 hineinzuragen und so das Verankerungselement 21c im Knochen 3 festzusetzen.

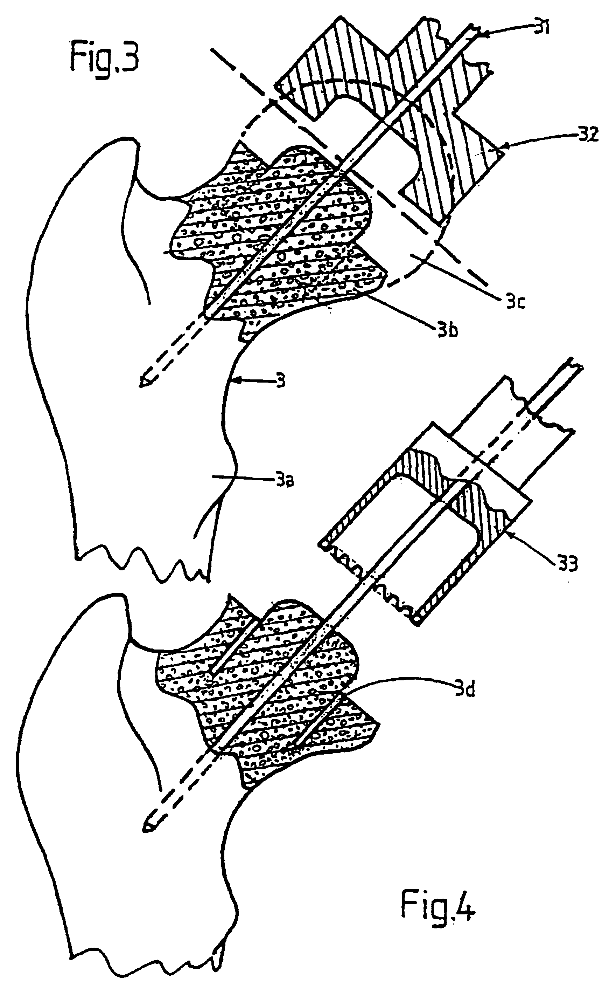

In den Figuren 1 bis 5, 7, 8 und 10 ist auch ein Femur 3 eines erwachsenen Menschen gezeichnet. Der Femur 3 besitzt einen länglichen, nur zum Teil gezeichneten Schaft 3a und einen Hals 3b, an den bei einem gesunden Knochen ein Gelenkkopf 3c anschließt, der in der Figur 3 nur noch andeutungsweise dargestellt wird. In der Figur 1 sind auch noch einige zur Blutversorgung des Knochens 3 dienende Blutgefäße 4 gezeichnet.

Anhand der Figuren 3 und 4 wird nun kurz erläutert, wie eine erfindungsgemäße Gelenkkopfprothese am Femur 3 befestigt wird. Dabei wird das in den Figuren 1 und 2 dargestellte Ausführungsbeispiel verwendet, das selbe Verfahren läßt sich natürlich auch in analoger Weise zur Befestigung der anderen Ausführungsformen der Erfindung am Femur 3 verwenden. In einem ersten Schritt wird der Gelenkkopf 3c mit einem operativen Eingriff freigelegt. Anschließend wird ein Führungsdraht 31 in den Kopf und den Hals 3b des Femurs 3 eingeführt. Darauf wird mit einer ersten Fräse 32, die von diesem Führungsdraht 31 geführt wird, der Gelenkkopf 3c so abgefräst, daß der Knochen eine zur Auflagefläche (1a und 1b) des Trägers 1 passende Form aufweist. Eine zweite Fräse 33, die ebenfalls vom Führungsdraht 31 geführt wird, kann nun den zu dem Verankerungselement passenden Ringspalt in den Femur fräsen, im dargestellten Beispiel also einen einzelnen zum Verankerungselement 1c komplementären Ringspalt 3d. Der Ringspalt 3d ist dabei so bemessen, daß das Verankerungselement 1c satt hineinpaßt. In einem nächsten Schritt wird der Führungsdraht 31 entfernt und der Träger 1 auf den Knochenstumpf aufgesetzt, so daß seine Auflageflächen 1a und 1b auf den Außenflächen des Knochenstumpfes aufsitzen. Beim Aufsetzen dringt das Verankerungselement 1c in den dafür vorgesehenen Ringspalt 3d ein und sorgt dafür, daß der Träger 1 sofort auf dem Knochen festsitzt, ohne daß er mit Knochenzement oder einem sonstigen Bindemittel befestigt werden muß. In seltenen Fällen, zum Beispiel bei sehr poröser Knochenstruktur, kann aber noch Knochenzement zur zusätzlichen Befestigung verwendet werden. In einem letzten Schritt wird dann eine geeignete Gelenkkappe 2 auf den Träger 1 aufgesetzt, die ebenfalls ohne Befestigungsmittel haftet. Die Abmessungen eines Trägers einer erfindungsgemäßen Prothese und damit natürlich auch die Abmessungen der Fräsen 32 und 22 sind nun so ausgestaltet, daß beim vorstehend dargelegten operativen Eingriff nur wenige Blutgefäße 4 des Femurs beschädigt werden und die Blutversorgung des nach der Operation übrigbleibenden Knochenstumpfes nicht wesentlich beeinträchtigt wird. Die Erhaltung der Blutgefäße 4 ermöglicht, daß der Knochen verheilen kann und daß neues Knochenmaterial gebildet wird. Nach einer gewissen Heilungsdauer füllt der bei der Operation durch Fräsen geformte Knochenstumpf zusammen mit dem nachgewachsenen Knochenmaterial die ganze Perforation 1d im Verankerungselement 1c und sorgt so dafür, daß der Träger 1 fest mit dem Knochen verwachsen ist.

Die Verankerungselemente können vorzugsweise einstückig mit dem Träger ausgebildet sein, es ist aber auch möglich, diese vom Träger lösbar auszubilden, so daß die Verankerungselemente in den Träger eingesetzt werden können, beispielsweise durch Einstecken oder Einschrauben. Dies ermöglicht eine Auswechselung der Verankerungselemente, so daß jeder Träger entsprechend den jeweiligen Notwendigkeiten mit unterschiedlichen Verankerungselementen bestückt werden kann. Die Länge der Verankerungselemente wird dabei so gewählt, daß sie auf der dem Schenkelhals gegenüberliegenden Seite nicht aus dem Femur austreten, dazu sollte diese Länge unter 50 mm liegen, vorzugsweise im Bereich zwischen 15 und 40 mm.

An einem Träger 1 können mehrere Verankerungselemente mit unterschiedlicher Länge und/oder unterschiedlichem Durchmesser angeordnet sein. Dies wird deutlich aus den Ausführungsbeispielen der Figuren 7 und 10. Beim Ausführungsbeispiel der Figur 7 sind zwei konzentrische Verankerungselemente 1c und 1i vorgesehen, das innere Verankerungselement 1c hat einen kleineren Durchmesser und eine größere Länge als das äußere Verankerungselement 1i. beim Ausführungsbeispiel der Figur 10 liegen die Verhältnisse ähnlich, in diesem Falle sind sogar drei innere Verankerungselemente 11c mit größerer Länge und deutlich kleinerem Durchmesser vorgesehen.

Die Verankerungselemente haben vorzugsweise einen kreisförmigen Querschnitt, es ist aber durchaus auch möglich, Verankerungselemente mit einem von der Kreisform abweichenden Querschnitt zu-verwenden, beispielsweise mit einem elliptischen oder ovalen Querschnitt.

Die Verankerungselemente weisen eine poröse Oberfläche - auf, um die Verankerung mit dem umgebenden Knochenmaterial zu ermöglichen. Diese Oberfläche kann beispielsweise bestehen aus Titan, aus Hydroxylapatit oder aus Calciumtriphosphat.

Wie das beispielsweise aus der Figur 1 ersichtlich ist, kann eine erfindungsgemäße Prothese zusammen mit einem in das Hüftbein 5 eingebrachten künstlichen Acetabulum 6 ein künstliches Hüftgelenk bilden. Dabei weist das Acetabulum 6 eine zur Gleitfläche 2a der Gelenkkappe 2 komplementäre Innenfläche 6a auf und besteht beispielsweise im wesentlichen aus einem biokompatiblen Kunststoff, etwa aus Polyethylen. Da die Gelenkkappe 2 einen Außendurchmesser aufweist, der kleiner ist als derjenige der Gelenkkappe eines natürlichen Hüftgelenkes, kann dabei das Acetabulum eine charakteristische Dicke von beispielsweise 10 mm aufweisen.

Ein mit einer erfindungsgemäßen Prothese ausgestattetes künstliches Hüftgelenk kann den Anforderungen an die Anatomie, die sich aus den Belastungen des Gelenkes ergeben, angepaßt werden. In der Figur 7 wird schematisch dargestellt, wie der Abstand zwischen Femur und Hüftbein durch Wahl des Kugelmittelpunktes M variiert werden kann. Im gezeichneten Beispiel kann zwischen zwei Gelenkkappen 2A und 2B mit Kugelmittelpunkten M_{A} und M_{B} gewählt werden, deren Position auf dem Träger sich um *h* unterscheidet, so daß der Abstand zwischen Femur und Hüftbein sich ebenfalls um die Größe *h* unterscheidet. Wie in der Figur 8 aufgezeigt, kann auch die laterale Position der Gelenkkappe ideal den Erfordernissen angepaßt werden. Das geschieht, indem, abweichend von den anhand der Figuren 1, 2 und 10 beschriebenen Prothesen, das Sackloch in der Gelenkkappe 2D, beispielsweise um einen Abstand s exzentrisch angebracht wird.

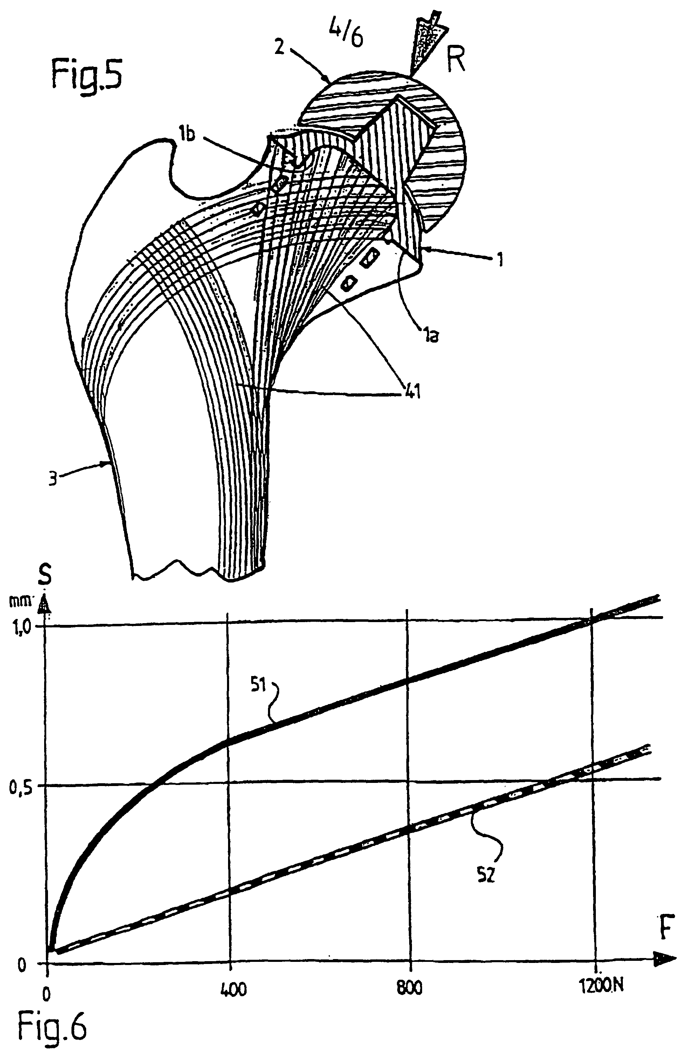

Anhand der Figuren 5 und 6 wird nun erklärt, warum eine erfindungsgemäße Gelenkkopfprothese eine im Vergleich zu herkömmlichen Schaftprothesen vorteilhafte Krafteinlenkung ermöglicht. In einem natürlichen Femur ist die Spongiosastruktur ideal auf die Belastungsverhältnisse abgestimmt, indem ihre Bälkchen entsprechend den in der Figur 5 schematisch eingezeichneten und mit 41 bezeichneten Zug- und Drucktrajektorien ausgerichtet sind. Eine am Gelenkkopf angreifende resultierende Kraft *R* weist nun im allgemeinen auch transversale, d.h. zu Kopfachse A rechtwinklige Kraftkomponenten auf. Wie das in der Figur 5 deutlich sichtbar ist, führt die wannenförmige Aussparung, die die innere Auflagefläche 1b des Trägers der erfindungsgemäßen Prothese bildet, dazu, daß auch diese Kraftkomponenten ideal von der Prothese auf den Knochen übertragen werden können. Die Figur 6 zeigt die an einem Femur eines erwachsenen Mannes gemessene Auslenkung *S* des Femurs in Funktion der am Gelenkkopf angreifenden Kraft *F* für den natürlichen Femur (Kurve 51) sowie für denselben Femur, bei dem anschließend an die erste Messung der Gelenkkopf durch eine konventionelle Schaftprothese-ersetzt wurde (Kurve 52). Dabei wird deutlich, daß der Differentialquotient dS/dF bei der am natürlichen Femur gemessenen im Vergleich zu der am künstlichen Femur gemessenen Kurve für kleine Kräfte sehr viel größer ist und dann in Funktion der Kraft bis zu einer Belastung von ca. 500 N stetig abnimmt. Der mit einer Schaftprothese versehene Femur hingegen weist einen schon für kleine Kräfte konstanten, von der Kraft unabhängigen Differentialquotienten auf. Für Kräfte, die größer als 500 N ist der Differentialquotient dann im wesentlichen für beide Proben konstant und gleich groß. Der im Vergleich zum natürlichen Femur deutlich kleinere Differentialquotient dS/dF des mit einer nicht erfindungsgemäßen Schaftprothese ausgestatteten Femurs hat zur Folge, daß sich nur eine beschränkte Federwirkung aus dem Hüftgelenk ergibt, wenn der Träger des Hüftgelenkes das Bein auf den Boden aufsetzt. Eine erfindungsgemäße Gelenkkopfprothese ist hingegen so ausgestaltet, daß sich durch die aufgrund der Geometrie des Trägers 1, 11 gegebene Krafteinleitung ein Verlauf der S(F)-Kurve ergibt, die mit derjenigen des natürlichen Femurs im wesentlichen übereinstimmt.

## Patentansprüche

1. Gelenkkopfprothese zur Befestigung an einem Knochen (3), insbesondere einem Femur, mit einem einen ringförmigen Abschnitt (1a, 11a) aufweisenen Träger (1, 11), einer ursprünglich separaten, fest mit diesem verbindbaren, eine kugelkalottenförmige Gleitfläche (2a, 12a) bildenden Gelenkkappe (2, 12) und mit mindestens einem vom Träger (1, 11) und von der Gleitfläche (2a, 12a) wegragenden hülsenförmigen Verankerungselement mit einer geraden Achse, wobei der Träger (1, 11) einen vom mindestens einem Verankerungselement (1c, 11c, 21c) wegragenden Vorsprung (1e, 11e), und die Gelenkkappe (2, 12) ein Sackloch (2c, 12c) aufweisen, in welches der Vorsprung (1e, 11e) bei zusammengesetzter Gelenkkopfprothese hineinragt und die Gelenkkappe (2, 12) festhält, **dadurch gekennzeichnet, daß** der Träger (1, 11) eine Auflagefläche mit einem wannenförmigen Abschnitt (1d, 11d) aufweist, den der ringförmige Abschnitt (1a, 11a) umschließt.

2. Gelenkkopfprothese nach Anspruch 1, **dadurch gekennzeichnet, daß** der Vorsprung (1e, 11e) des Trägers (1, 11) konisch ist und sich vom mindestens einen Verankerungselement (1c, 11c, 21c) weg verjüngt, wobei der Vorsprung (1e, 11e) eine Achse definiert, die zu einer von der Gelenkkappe (2, 12) definierten Kopfachse (A) parallel verläuft und zu der die Konusfläche einen Winkel von höchstens 10° bildet.

3. Gelenkkopfprothese nach Anspruch 2, **dadurch gekennzeichnet, daß** die Achse des Vorsprunges (1e, 11e) und die Kopfachse zusammenfallen.

4. Gelenkkopfprothese nach einem der Ansprüche 1 bis 3, **dadurch gekennzeichnet, daß** die Gelenkkappe (2, 12) aus Keramik, vorzugsweise aus Zirkoniumoxyd oder aus Aluminiumoxydkeramik, aus einer Stahllegierung oder aus Kunststoff ausgebildet ist.

5. Gelenkkopfprothese nach einem der voranstehenden Ansprüche, **dadurch gekennzeichnet, daß** der Träger (1, 11) und das bzw. jedes Verankerungselement (1c, 11c, 21c) zusammen aus einem einstückigen Körper bestehen.

6. Gelenkkopfprothese nach einem der voranstehenden Ansprüche, **dadurch gekennzeichnet, daß** die Achse des Verankerungselementes (1c, 11c, 21c) oder der Verankerungselemente (1c, 11c, 21c) parallel zu einer von der Gelenkkappe (2, 12) definierten Köpfachse (A) verläuft.

7. Gelenkkopfprothese nach Anspruch 6, **dadurch gekennzeichnet, daß** die Achse des Verankerungselementes (1c, 11c, 21c) mit der Kopfachse (A) zusammenfällt.

8. Gelenkkopfprothese nach einem der voranstehenden Ansprüche, **dadurch gekennzeichnet, daß** das Verankerungselement (1c, 11c, 21c) die Form eines perforierten Hohlzylinders mit einem offenen Ende aufweist.

9. Gelenkkopfprothese nach einem der voranstehenden Ansprüche, **dadurch gekennzeichnet, daß** das Verankerungselement (21c) außen und/oder innen Längsrippen (21d) trägt.

10. Gelenkkopfprothese nach einem der voranstehenden Ansprüche, **dadurch gekennzeichnet, daß** das oder die Verankerungselemente (1c, 11c, 21c) im Querschnitt kreisförmig ausgebildet sind.

11. Gelenkkopfprothese nach einem der Ansprüche 1 bis 9, **dadurch gekennzeichnet, daß** das oder die Verankerungselemente (1c, 11c, 21c) im Querschnitt von der Kreisform abweichend, insbesondere elliptisch oder oval ausgebildet sind.

12. Gelenkkopfprothese nach einem der Ansprüche 1 bis 4 oder 6 bis 11, **dadurch gekennzeichnet, daß** das Verankerungselement oder die Verankerungselemente (1c, 11c, 21c) lösbar am Träger (1, 11) gehalten sind.

13. Gelenkkopfprothese nach einem der voranstehenden Ansprüche, **dadurch gekennzeichnet, daß** die Verankerungselemente (1c, 11c, 21c)mit einer porösen Beschichtung versehen sind.

14. Gelenkkopfprothese nach Anspruch 13, **dadurch gekennzeichnet, daß** die poröse Beschichtung aus Titan, aus Hydroxylapatit oder aus Tricalciumphosphat besteht.

15. Gelenkkopfprothese nach einem der voranstehenden Ansprüche, **dadurch gekennzeichnet, daß** das oder die Verankerungselemente (1c, 1i, 11c, 11i, 21c) kürzer als 50 mm sind.

16. Gelenkkopfprothese nach Anspruch 15, **dadurch gekennzeichnet, daß** die Verankerungselemente (1c, 1i, 11c, 11i, 21c) eine Länge von 15 bis 40 mm aufweisen.

17. Gelenkkopfprothese nach einem der voranstehenden Ansprüche, **dadurch gekennzeichnet, daß** mehrere Verankerungselemente (1c, 1i; 11c, 11i) mit unterschiedlicher Länge und/oder unterschiedlichem Durchmesser an einem Träger (1, 11) angeordnet sind.

18. Gelenkkopfprothese nach einem der voranstehenden Ansprüche, **dadurch gekennzeichnet, daß** an einem Träger (1, 11) mindestens zwei konzentrisch zueinander angeordnete Verankerungselemente (1c, 1i, 11c, 11i) angeordnet sind.

19. Gelenkkopfprothese nach einem der voranstehenden Ansprüche, **dadurch gekennzeichnet, daß** der Auflageflächenrand (1h, 11h) der Auflagefläche den Gleitflächenrand (2e, 12e) der Gleitfläche (2a, 12a) umschließt.

20. Gelenkkopfprothese nach Anspruch 19, **dadurch gekennzeichnet, daß** die Gleitfläche (2a, 12a) einen Kugeldurchmesser dₖ definiert und daß der Auflageflächenrand (1h) kreisförmig ist und einen Durchmesser dₐ hat, der größer als der Kugeldurchmesser dₖ ist.

21. Gelenkkopfprothese nach Anspruch 20, **dadurch gekennzeichnet, daß** die Gleitfläche (2a, 12a) mehr als eine Halbkugelfläche bildet, wobei der Gleitflächenrand (2e, 12e) kreisförmig ist und einen Durchmesser d_{g} hat, der kleiner als der Kugeldurchmesser dₖ ist.

22. Gelenkkopfprothese nach Anspruch 20 oder 21, **dadurch gekennzeichnet, daß** der Kugeldurchmesser dₖ 50 % bis 95 % von dₐ beträgt.

23. Gelenkkopfprothese nach einem der voranstehenden Ansprüche, **dadurch gekennzeichnet, daß** der Auflageflächenrand (1h) kreisförmig ist.

24. Gelenkkopfprothese nach einem der Ansprüche 1 bis 23, **dadurch gekennzeichnet, daß** der wannenförmige Abschnitt (1b, 11b) der Auflagefläche eine vom Auflageflächenrand (1h) aus parallel zur Kopfachse (A) zu einer tiefsten Stelle des wannenförmigen Abschnittes (1b, 11b) gemessene Tiefe t hat, die mindestens 12 % des Durchmessers dₐ des Auflageflächenrandes (1h) beträgt.

25. Gelenkkopfprothese nach Anspruch 24, **dadurch gekennzeichnet, daß** die Tiefe t 15 bis 25 % des Durchmessers dₐ des Auflageflächenrandes (1h) beträgt.

26. Gelenkkopfprothese nach einem der voranstehenden Ansprüche, **dadurch gekennzeichnet, daß** der ringförmige Abschnitt (1a, 11a) der Auflagefläche mit der Kopfachse (A) einen Winkel von 80° bis 100° bildet.

27. Gelenkkopfprothese nach einem der voranstehenen Ansprüche, **dadurch gekennzeichnet, daß** die Breite des ringförmigen Abschnittes mindestens 12 % des Durchmessers dₐ des Auflageflächenrandes (1h) beträgt.

28. Bausatz mit einer Gelenkkopfprothese nach einem der voranstehenden Ansprüche, **dadurch gekennzeichnet, daß** er zusätzlich zur genannten Gelenkkappe (2, 12) mindestens noch eine andere Gelenkkappe (2, 12) aufweist, daß die Gelenkkappen (2, 12) derart ausgebildet sind, daß wahlweise eine von ihnen am Träger (1, 11) befestigbar ist und daß die Gelenkkappen (2, 12) unterschiedliche Formen und/oder Maße aufweisen.

29. Bausatz nach Anspruch 28, **dadurch gekennzeichnet, daß** bei einer Gelenkkappe (2, 12) das Sackloch (2c, 12c) koaxial zur Kopfachse (A) und bei einer anderen Gelenkkappe (2, 12) exzentrisch zur Kopfachse (A) angeordnet ist.

30. Bausatz nach einem der Ansprüche 28 oder 29, **dadurch gekennzeichnet, daß** Gelenkkappen (2, 12) vorhanden sind, deren Gleitflächen (2a, 12a) unterschiedliche Kugeldurchmesser definieren.

31. Bausatz nach einem der Ansprüche 28 bis 30, **dadurch gekennzeichnet, daß** Gelenkkappen (2, 12) vorhanden sind, deren Gleitflächen (2a, 12a) Kugelzentren definieren, die bei zusammengesetzter Gelenkkopfprothese verschiedene Abstände vom Auflageflächenrand (1h) haben.

## Claims

1. A condyle prosthesis for attachment to a bone (3), more particularly a femur, comprising a support (1, 11) having an annular portion (1a, 11a), an originally separate joint cap (2, 12) which may be fixedly connected to the said support and forms a spherical cap-shaped sliding surface (2a, 12a), and at least one sleeve-shaped anchoring element with a straight axis, the said element projecting away from the support (1, 11) and from the sliding surface (2a, 12a), the support (1, 11) having a projection (1e, 11e) projecting away from the at least one anchoring element (1c, 11c, 21c), and the joint cap (2, 12) having a blind hole (2c, 12c), into which the projection (1e, 11e) projects when the condyle prosthesis is assembled and secures the joint cap (2, 12), **characterised in that** the support (1, 11) has a mounting surface comprising a trough-shaped portion (1d, 11d) [sic, recte (1b, 11b)] which the annular portion (1a, 11a) encloses.

2. A condyle prosthesis according to Claim 1, **characterised in that** the projection (1e, 11e) of the support (1, 11) is conical and tapers away from the at least one anchoring element (1c, 11c, 21c), the projection (1e, 11e) defining an axis which extends parallel to a caput axis (A) defined by the joint cap (2, 12) and with which the conical surface forms an angle of a maximum of 10°.

3. A condyle prosthesis according to Claim 2, **characterised in that** the axis of the projection (1e, 11e) and the caput axis coincide.

4. A condyle prosthesis according to one of Claims 1 to 3, **characterised in that** the joint cap (2, 12) is made from ceramics, preferably zirconium oxide or aluminium oxide ceramics, a steel alloy or plastics material.

5. A condyle prosthesis according to one of the preceding claims, **characterised in that** the support (1, 11) and the, or each, anchoring element (1c, 11c, 21c) together consist of a single-piece body.

6. A condyle prosthesis according to one of the preceding claims, **characterised in that** the axis of the anchoring element (1c, 11c, 21 c) or anchoring elements (1c, 11c, 21c) extends parallel to a caput axis (A) defined by the joint cap (2, 12).

7. A condyle prosthesis according to Claim 6, **characterised in that** the axis of the anchoring element (1c, 11, 21c) coincides with the caput axis (A).

8. A condyle prosthesis according to one of the preceding claims, **characterised in that** the anchoring element (1c, 11c, 21 c) has the shape of a perforated hollow cylinder with an open end.

9. A condyle prosthesis according to one of the preceding claims, **characterised in that** the anchoring element (21 c) bears longitudinal ribs (21d) on the outside and/or the inside.

10. A condyle prosthesis according to one of the preceding claims, **characterised in that** the anchoring element or elements (1c, 11c, 21c) is/are designed so as to be circular in cross-section.

11. A condyle prosthesis according to one of Claims 1 to 9, **characterised in that** the anchoring element or elements (1c, 11c, 21c) is/are designed so as to differ from the circular shape in cross-section, more particularly so as to be elliptical or oval.

12. A condyle prosthesis according to one of Claims 1 to 4 or 6 to 11, **characterised in that** the anchoring element or the anchoring elements (1c, 11c, 21c) is/are detachably held on the support (1, 11).

13. A condyle prosthesis according to one of the preceding claims, **characterised in that** the anchoring elements (1c, 11c, 21c) are provided with a porous coating.

14. A condyle prosthesis according to Claim 13, **characterised in that** the porous coating consists of titanium, hydroxyl apatite or tricalcium phosphate.

15. A condyle prosthesis according to one of the preceding claims, **characterised in that** the anchoring element or elements (1c, 1i, 11c, 11i, 21c,) is/are shorter than 50 mm.

16. A condyle prosthesis according to Claim 15, **characterised in that** the anchoring elements (1c, 1i, 11c, 11i, 21c) have a length of 15 to 40 mm.

17. A condyle prosthesis according to one of the preceding claims, **characterised in that** several anchoring elements (1c, 1i; 11c, 11i) with a different length and/or a different diameter are arranged on a support (1, 11).

18. A condyle prosthesis according to one of the preceding claims, **characterised in that** at least two anchoring elements (1c, 1i, 11c, 11i) are arranged on a support (1, 11), arranged concentrically to one another.

19. A condyle prosthesis according to one of the preceding claims, **characterised in that** the mounting surface margin (1h, 11h) of the mounting surface encloses the sliding surface margin (2e, 12e) of the sliding surface (2a, 12a).

20. A condyle prosthesis according to Claim 19, **characterised in that** the sliding surface (2a, 12a) defines a ball diameter dₖ, and **in that** the mounting surface margin (1h) is circular and has a diameter dₐ which is larger than the ball diameter dₖ.

21. A condyle prosthesis according to Claim 20, **characterised in that** the sliding surface (2a, 12a) forms more than a hemispherical surface, the sliding surface margin (2e, 12e) being circular and having a diameter dg which is smaller than the ball diameter dₖ.

22. A condyle prosthesis according to Claim 20 or 21, **characterised in that** the ball diameter dₖ is 50 % to 95 % of dₐ.

23. A condyle prosthesis according to one of the preceding claims, **characterised in that** the mounting surface margin (1h) is circular.

24. A condyle prosthesis according to one of Claims 1 to 23, **characterised in that** the trough-shaped portion (1b, 11b) of the mounting surface has a depth t measured from the mounting surface margin (1h) parallel to the caput axis (A) to a deepest point of the trough-shaped portion (1b, 11b), which depth is at least 12 % of the diameter dₐ of the mounting surface margin (1h).

25. A condyle prosthesis according to Claim 24, **characterised in that** the depth t is 15 to 25 % of the diameter dₐ of the mounting surface margin (1h).

26. A condyle prosthesis according to one of the preceding claims, **characterised in that** the annular portion (1a, 11a) of the mounting surface forms an angle of 80° to 100° with the caput axis (A).

27. A condyle prosthesis according to one of the preceding claims, **characterised in that** the width of the annular portion is at least 12 % of the diameter dₐ of the mounting surface margin (1h).

28. A kit comprising a condyle prosthesis according to one of the preceding claims, **characterised in that**, in addition to the said joint cap (2, 12), it has at least one other joint cap (2, 12), **in that** the joint caps (2, 12) are designed in such a way that, optionally, one of them is attachable to the support (1, 11), and **in that** the joint caps (2, 12) have different shapes and/or dimensions.

29. A kit according to Claim 28, **characterised in that** in one joint cap (2, 12) the blind hole (2c, 12c) is arranged coaxially to the caput axis (A) and in another joint cap (2, 12) it is arranged eccentrically of the caput axis (A).

30. A kit according to one of Claims 28 or 29, **characterised in that** joint caps (2, 12) are available, the sliding surfaces (2a, 12a) of which define different ball diameters.

31. A kit according to one of Claims 28 to 30, **characterised in that** joint caps (2, 12) are available, the sliding surfaces (2a, 12a) of which define centres of balls which are differently spaced from the mounting surface margin (1h) when the condyle prosthesis is assembled.

## Revendications

1. Prothèse de tête d'articulation destinée à être fixée à un os (3), notamment un fémur, comprenant un support (1, 11) présentant un tronçon de forme annulaire (1a, 11a), une coiffe d'articulation (2, 12) formant une surface de glissement (2a, 12a) en forme de calotte sphérique, initialement séparée du support et pouvant être reliée de manière fixe à celui-ci, et au moins un élément d'ancrage en forme de douille qui présente un axe rectiligne et fait saillie en s'écartant du support (1, 11) et de la surface de glissement (2a, 12a), le support (1, 11) présentant une protubérance (1e, 11e) en saillie et s'éloignant du dit au moins un élément d'ancrage (1c, 11c, 21c), et la coiffe d'articulation (2, 12) présentant un trou borgne (2c, 12c) dans lequel vient s'engager la protubérance (1e, 11e) en y maintenant de manière fixe la coiffe d'articulation (2, 12), lorsque la prothèse de tête d'articulation est assemblée,
**caractérisée en ce que** le support (1, 11) présente une surface d'appui avec un tronçon en forme de cuvette (1b, 11b) entouré par ledit tronçon de forme annulaire (1a, 11a).

2. Prothèse de tête d'articulation selon la revendication 1, **caractérisée en ce que** la protubérance (1e, 11e) du support (1, 11) est conique, et se rétrécit en s'éloignant dudit au moins un élément d'ancrage (1c, 11c, 21c), la protubérance (1e, 11e) définissant un axe qui s'étend parallèlement à un axe de tête (A) défini par la coiffe d'articulation (2, 12), et par rapport auquel la surface conique forme un angle d'au plus 10°.

3. Prothèse de tête d'articulation selon la revendication 2, **caractérisée en ce que** l'axe de la protubérance (1e, 11e) et l'axe de tête sont confondus.

4. Prothèse de tête d'articulation selon l'une des revendications 1 à 3, **caractérisée en ce que** la coiffe d'articulation (2, 12) est réalisée en céramique, de préférence en oxyde de zirconium ou en une céramique d'oxyde d'aluminium, ou en un alliage d'acier ou en matière plastique.

5. Prothèse de tête d'articulation selon l'une des revendications précédentes, **caractérisée en ce que** le support (1, 11) et l'élément d'ancrage ou chaque élément d'ancrage (1c, 11c, 21c), sont constitués en commun par un corps d'un seul tenant.

6. Prothèse de tête d'articulation selon l'une des revendications précédentes, **caractérisée en ce que** l'axe de l'élément d'ancrage (1c, 11c, 21c) ou des éléments d'ancrage (1c, 11c, 21c) s'étend parallèlement à un axe de tête (A) défini par la coiffe d'articulation (2, 12).

7. Prothèse de tête d'articulation selon la revendication 6, **caractérisée en ce que** l'axe de l'élément d'ancrage (1c, 11c, 21c) est confondu avec l'axe de tête (A).

8. Prothèse de tête d'articulation selon l'une des revendications précédentes, **caractérisée en ce que** l'élément d'ancrage (1c, 11c, 21c) présente la forme d'un cylindre creux perforé présentant une extrémité ouverte.

9. Prothèse de tête d'articulation selon l'une des revendications précédentes, **caractérisée en ce que** l'élément d'ancrage (21c) porte, sur l'extérieur et/ou l'intérieur, des nervures longitudinales (21d).

10. Prothèse de tête d'articulation selon l'une des revendications précédentes, **caractérisée en ce que** l'élément d'ancrage ou les éléments d'ancrage (1c, 11c, 21c) sont d'une configuration de forme circulaire en section transversale.

11. Prothèse de tête d'articulation selon l'une des revendications 1 à 9, **caractérisée en ce que** l'élément d'ancrage ou les éléments d'ancrage (1c, 11c, 21c) sont, en section transversale, d'une configuration s'écartant de la forme circulaire, à savoir notamment d'une configuration de forme elliptique ou ovale.

12. Prothèse de tête d'articulation selon l'une des revendications 1 à 4 ou 6 à 11, **caractérisée en ce que** l'élément d'ancrage ou les éléments d'ancrage (1c, 11c, 21c) sont maintenus de manière amovible sur le support (1, 11).

13. Prothèse de tête d'articulation selon l'une des revendications précédentes, **caractérisée en ce que** les éléments d'ancrage (1c, 11c, 21c) sont pourvus d'un revêtement poreux.

14. Prothèse de tête d'articulation selon la revendication 13, **caractérisée en ce que** le revêtement poreux est réalisé en titane, en hydroxlapatite ou en tricalciumphosphate.

15. Prothèse de tête d'articulation selon l'une des revendications précédentes, **caractérisée en ce que** l'élément d'ancrage ou les éléments d'ancrage (1c, 1i, 11c, 11i, 21c) sont plus courts que 50 mm.

16. Prothèse de tête d'articulation selon la revendication 15, **caractérisée en ce que** l'élément d'ancrage ou les éléments d'ancrage (1c, 1i, 11c, 11i, 21c) présentent une longueur de 15 à 40 mm.

17. Prothèse de tête d'articulation selon l'une des revendications précédentes, **caractérisée en ce que** plusieurs éléments d'ancrage (1c, 1i, 11c, 11i) d'une longueur différente et/ou d'un diamètre différent sont disposés sur un support (1, 11).

18. Prothèse de tête d'articulation selon l'une des revendications précédentes, **caractérisée en ce que** sur un support (1, 11) sont disposés au moins deux éléments d'ancrage (1c, 1i, 11c, 11i) placés concentriquement l'un par rapport à l'autre.

19. Prothèse de tête d'articulation selon l'une des revendications précédentes, **caractérisée en ce que** le bord de surface d'appui (1h, 11h) de la surface d'appui, entoure le bord de surface de glissement (2e, 12e) de la surface de glissement (2a, 12a).

20. Prothèse de tête d'articulation selon la revendication 19, **caractérisée en ce que** la surface de glissement (2a, 12a) définit un diamètre de sphère dₖ, et **en ce que** le bord de surface d'appui (1h) est de forme circulaire et a un diamètre dₐ qui est supérieur au diamètre de sphère dₖ.

21. Prothèse de tête d'articulation selon la revendication 20, **caractérisée en ce que** la surface de glissement (2a, 12a) forme une surface de plus d'une demi-sphère, le bord de surface de glissement (2e, 12e) étant de forme circulaire et présentant un diamètre dg qui est inférieur au diamètre de sphère dₖ.

22. Prothèse de tête d'articulation selon la revendication 20 ou 21, **caractérisée en ce que** le diamètre de sphère dₖ vaut de 50% à 95% de dₐ.

23. Prothèse de tête d'articulation selon l'une des revendications précédentes, **caractérisée en ce que** le bord de surface d'appui (1h) est de forme circulaire.

24. Prothèse de tête d'articulation selon l'une des revendications 1 à 23, **caractérisée en ce que** le tronçon en forme de cuvette (1b, 11b) de la surface d'appui présente une profondeur t, mesurée à partir du bord de surface d'appui (1h), parallèlement à l'axe de tête (A), jusqu'à l'endroit le plus profond du tronçon en forme de cuvette (1b, 11b), qui vaut au moins 12% du diamètre dₐ du bord de surface d'appui (1h).

25. Prothèse de tête d'articulation selon la revendication 24, **caractérisée en ce que** la profondeur t vaut de 15 à 25% du diamètre dₐ du bord de surface d'appui (1h).

26. Prothèse de tête d'articulation selon l'une des revendications précédentes, **caractérisée en ce que** le tronçon de forme annulaire (1a, 11a) de la surface d'appui forme avec l'axe de tête (A), un angle de 80° à 100°.

27. Prothèse de tête d'articulation selon l'une des revendications précédentes, **caractérisée en ce que** la largeur du tronçon de forme annulaire vaut au moins 12% du diamètre dₐ du bord de surface d'appui (1h) .

28. Jeu de pièces comprenant une prothèse de tête d'articulation selon l'une des revendications précédentes, **caractérisé en ce qu'**il comprend en plus de la coiffe d'articulation (2, 12) citée, au moins une autre coiffe d'articulation (2, 12) supplémentaire, **en ce que** les coiffes d'articulation (2, 12) sont conçues de façon à pouvoir fixer l'une d'elles au choix sur le support (1, 11), et **en ce que** les coiffes d'articulation (2, 12) présentent des formes et/ou des dimensions différentes.

29. Jeu de pièces selon la revendication 28, **caractérisé en ce que** dans une coiffe (2, 12) le trou borgne (2c, 12c) est disposé coaxialement à l'axe de tête (A), et dans l'autre coiffe d'articulation (2, 12) il est disposé de manière excentrée par rapport à l'axe de tête (A).

30. Jeu de pièces selon l'une des revendications 28 ou 29, **caractérisé en ce qu'**il comprend des coiffes d'articulation (2, 12) dont les surfaces de glissement (2a, 12a) définissent des diamètres de sphère différents.

31. Jeu de pièces selon l'une des revendications 28 à 30, **caractérisé en ce qu'**il comprend des coiffes d'articulation (2, 12) dont les surfaces de glissement (2a, 12a) définissent des centres de sphères, qui présentent des distances différentes au bord de surface d'appui (1h) pour une prothèse de tête d'articulation assemblée.
